# EUROPEAN PATENT APPLICATION

(11) **EP 1 431 398 A1**
(43) Date of publication of application: **23.06.2004**
(21) Application number: 02028582.1
(22) Date of filing: 20.12.2002
(51) Int. Cl.: C12Q 1/68

(54) **A method for detecting in a mixture an amount of analytes**

(71) Applicant: Evotec OAI AG, 22525 Hamburg (DE)
(72) Inventor: Hinnah, Silke, 22457 Hamburg (DE); Lambrü, Dagmar, 29328 Fassberg (DE); Dröge, Sonja, 25335 Elmshorn (DE); Jäger, Stefan, 20251 Hamburg (DE); Gall, Karsten, 27616 Lunestedt (DE); Stürmer, Werner, Dr. c/o Altana Pharma AG, 78467 Konstanz (DE); Schäfer, Michaela, Dr. c/o Altana Pharma AG, 78467 Konstanz (DE)
(74) Representative: Meyers, Hans-Wilhelm, Dr.Dipl.-Chem.

(57) **Abstract**

A method for detecting in a mixture an amount of analytes with probes specifically interacting with the analytes
- the probe having a first reporter unit reporting of binding of the probe to the analyte,
- the analyte is bound to a solid support,
- the solid support is labelled with a second reporter unit,
- the method comprising the steps of
- contacting a sample containing the analyte with the solid support,
- contacting the analyte with the probe having the first reporter unit,
- measuring the binding of the probe with the analyte via the first reporter unit, and
considering a signal due to the second reporter unit which is labelling the support.

A homogeneous assay for detecting analytes in a sample by using
- detection oligo nucleotides being labelled with a dye which reports binding of the deletion oligo nucleotide (DO) to the analytes,
- capture oligo nucleotides bound to a solid support, the capture oligo nucleotides (CO) concentrating the analyte at the surface of the solid support,
- the method comprising the steps of contacting a sample with the detection oligo nucleotides and the solid support, having capture oligo nucleotides and measuring the amount of detection oligo nucleotides bound to the analyte which is concentrated at the surface of the solid support.

## Description

The present invention pertains to a method for detecting in a mixture an amount of analytes with probes specifically interacting with the analytes.

The quantitative analysis of nucleic acids is an important tool in the molecular biology laboratory. Examples are genetic tests, virus diagnostics, analysis of polymorphisms. To date a number of DNA/RNA quantification systems have been developed, all of them involving amplification steps either performing exponentially (realised by Polymerase chain reaction (PCR), which is based on a specific, multiple turnover replication of the nucleic acid section to be identified) or linearly (realised by enzymatic turnover after hybridisation to a specific nucleic acid section). In most cases washing steps are indispensable, which make miniaturisation time consuming and difficult.

An object of the present invention is the detection of low concentrations of analytes, such as nucleic acids in high throughput screening which method allows a direct quantification of the analytes in a homogeneous assay.

The object of the present invention is accomplished by a method for detecting in a mixture an amount of analytes with probes specifically interacting with the analytes
- the probe having a first reporter unit reporting of binding of the probe to the analyte,
- the analyte is bound to a solid support,
- the solid support is labelled with a second reporter unit,
- the method comprising the steps of
- contacting a sample containing the analyte with the solid support,
- contacting the analyte with the probe having the first reporter unit,
- measuring the binding of the probe with the analyte via the first reporter unit, and
- considering of a signal due to the second reporter unit which is labelling the support.

The method of the invention is advantageous since no washing steps and in the case of RNA or DNA no enzymatic amplification of the signal is necessary. In the case of detection of nucleic acids a direct detection of the nucleic acids by detection oligonucleotides becomes possible. Furthermore, well established confocal detection systems and devices become applicable. The quantifying signal, which is e.g. fluorescent light, is directly linked to the analytes, omitting any amplifying turnover step. This makes the quantifying system easy to handle, extremely robust and amenable to high troughput screening. Additional features are a dynamic range of 3 orders of magnitude, variability smaller than 15 % and a reaction volume of about 25 µL, while reading and evaluating a 384 plate within about 10 minutes.

In a second aspect of the invention, a homogeneous assay is provided for detecting analytes in a sample by using
- detection oligo nucleotides being labelled with a dye which reports binding of the detection oligo nucleotide (DO) to the analytes,
- capture oligo nucleotides bound to a solid support, the capture oligo nucleotides (CO) concentrating the analyte at the surface of the solid support,
- the method comprising the steps of contacting the sample with the detection oligo nucleotides and the solid support having capture oligo nucleotides, and measuring the amount of detection oligo nucleotides bound to the analyte which is concentrated at the surface of the solid support.

In the method according to the second aspect preferably a quencher oligo nucleotide is added in order to reduce the fluorescence background resulting in particular from detection oligo nucleotides not bound to the analyte.

Good results are in particular achieved when the solid support is labelled with a dye different from the dye of the detection oligo nucleotide. In particular, the dyes are luminescent dyes and the analytes are proteins or nucleic acids.

Figure 1 shows the assay principle with the example of the detection of mRNA as target bound to a streptavidin coated bead via biotinylated capture oligonucleotides.

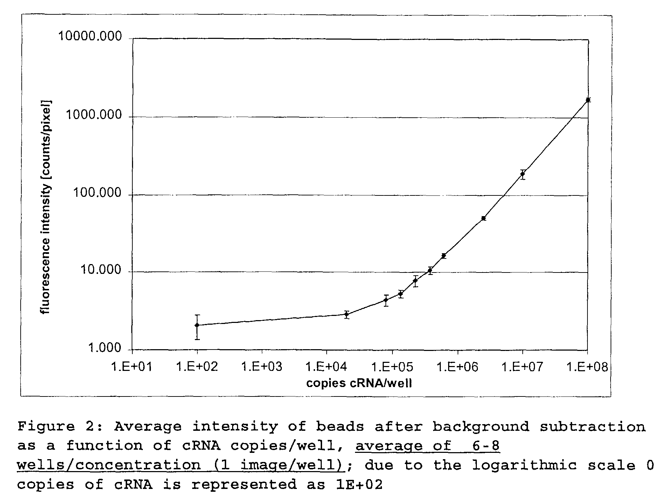

Figure 2 shows the linearity of RNA detection and detection limit.

In the case that the probe having the first reporter unit is a fluorescent probe and there are only a few analytes present it normally happens that the probe is present in an excess. Non-bound probe then emits fluorescent light which may cause a lowering of the sensitivity of the measurement. Therefore, it is advantageous to add a quencher of a fluorescence of the first reporter unit and to reduce the background.

Additionally, the background signal can be eliminated by mathematical methods. For example, the background signal is quantified in sufficient distance of the solid support and subtracted from the first reporter unit signal.

In another embodiment of the invention the first and/or the second reporter unit is a dye. Preferably the first and/or second reporter unit comprises a fluorescent or chemiluminescent dye. It is advantageous that the dye of the first reporter unit is different from the dye of the second reporter unit.

The probe having the first reporter unit is used for detecting the actual analyte, whereas the second reporter unit serves as marker for the solid support itself to which the analyte is bound, if present. Thus the second reporter unit allows the localisation of the solid support and to improve the accuracy of the measurement. In a preferred embodiment the reporter units are dyes having different absorption maxima and/or if they are fluorescent dyes different emission spectra. The skilled person readily understands how to chose the dyes according to the fluorescent filters in the measuring device which filters separate the two dyes.

According to the invention it becomes possible to determine analytes such as proteins, nucleic acids, and other substances such as secreted products e.g. originated from cells.

In particular, the analyte comprises two binding sites, one for the capture unit and another one for the probe. Binding of the probe to the analyte may be provided by means of hybridisation, antibodies or aptamers.

Typically, according to the invention the solid supports are beads, in particular suspendable beads, cells, bacteria.

In a convenient embodiment of the invention streptavidin coated polystyrene beads (from Spherotech, Libertyville, IL 60048) are used having a diameter of about 6 µm.

According to the invention the analyte is bound to the support by a capture unit. The capture unit of the invention may comprise a first portion bound to the support and a second portion capable of binding the analyte.
Each support may comprise a multitude of capture units.

The capture unit may be one single molecule or may consist of two or more molecules, wherein a respective terminal molecule would represent the portion capable of binding the support and a second portion capable of binding the analyte.

In particular, the first portion of the capture unit is covalently bound to the support. The solid support may be coated with carboxyl functional group which may react with an amino functional group of the capture portion thus forming a covalent amide bond.

In an embodiment of the invention the first portion of the capture unit is bound to the support by affinity interaction. Affinity interaction is mediated for example by an antigen and an antibody binding to the antigen. Another example of an affinity interaction is the biotin/streptavidin system. In one embodiment of the invention, the first portion of the capture unit is biotinyl and the support is at least partially coated with streptavidin.

It is also possible to coat the support with protein A and bind antibodies of the IgG-type to it, which specifically recognises a structure of the capture unit representing the first portion, binding the capture unit via the antibody to the support. The second portion of such capture unit then binds the analyte.

According to the invention the analyte may be RNA such as mRNA and the second portion of the capture unit may be an oligo nucleotide hybridizing with the mRNA. Typically, the probe comprises an oligo nucleotide hybridizing with the nucleic acid analyte. Preferably, the probe is an oligo nucleotide hybridizing with the oligo nucleotide analyte, the probe having covalently bound a fluorescent dye as a reporter. The following dyes may be used Fluorescein, Rhodamine Green, Tetramethylrhodamine (TAMRA), Cy3, rhodamine-6-G, latter is particularly preferred.

The addition of a quencher can be advantageous. If low concentrations of analytes have to be quantified a large number of unbound probe e.g. detection oligonucleotides are present. This leads to increase of background signal which causes lowering of the detection limit.

According to the invention, the fluorescence of unbound probes/first reporter unit is quenched with a quencher oligo nucleotide.

According to the invention, the second reporter unit is bound to the support covalently or by affinity interaction, e.g. the second reporter unit is biotinylated and is attached to the support via the streptavidin which is linked to the support.

The second reporter unit is preferably labelled with a dye selected from the group consisting of MR 121, Cy5, Texas Red or EvoBlue30.

The method of the invention is preferably used in High Throughput Screening (HTS).

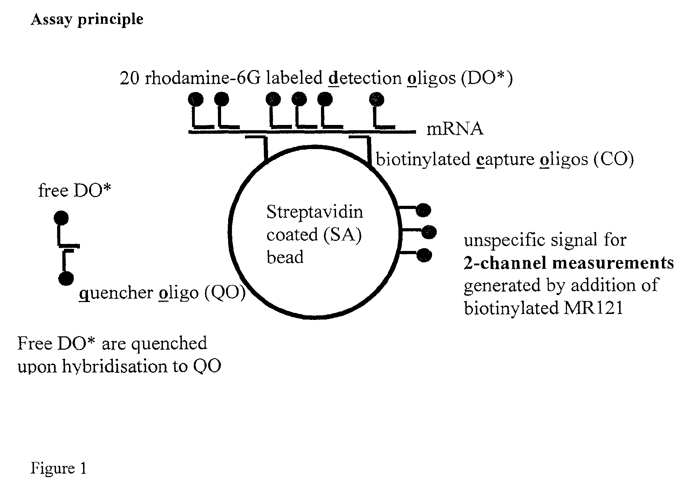

The invention is further described exemplary. The assay principle is further disclosed and explained in figure 1. A basic principle can be regarded as filtering and fixing freely diffusing analytes, such as RNA/DNA from a 3-dimensional assay volume into quasi 2-dimensional detection volume. Figure 1 shows a preferred assay formate in which streptavidin coated beads are labeled with biotinylated MR121 dye. This dye represents the second reporter unit according to the invention. In the specific example it is a red dye. The surface of the streptavidin coated bead is partially occupied by biotinylated capture oligo nucleotides consisting of eight different nucleotides. To this is bound the analyte, in the present case mRNA. It was obtained after stimulation of A549 lung epithelium cells by a cytokin mixture. To the complex, i.e. mRNA immobilised via the capture oligos (CO) to the support, an oligo detection oligonucleotide (DO*) is bound by hybridisation. This represents the probe which in this case is labeled with a green fluorescent dye, rhodamine-6-G. In order to improve the detection limit free DO* are quenched by quencher oligonucleotides (QO). The quencher oligonucleotide should have a complementary sequence to those of DO* but preferably having a slightly shorter length. For example, if the detection oligonucleotides have about 20 nucleotides the quencher oligo should have about 15 bases.

The mRNA to be analysed will be captured by capture oligos which preferably satisfy the conditions:
1. Recognition sequence of the CO (preferably about 20 basis) is complementary to the analyte sequence.
2. A region different from the recognition sequence of the CO should include a link which binds to the beads. The link can be realised by a biotin molecule which captures the CO to a streptavidin coated bead. Capturing can also be realised by direct covalent link of the CO to the bead or by any other appropriate means of binding the CO to the bead. In this manner the analyte mRNA will be captured by hybridising to the CO which binds to the beads.

Subsequently or in parallel to the capturing process the analyte mRNA will be marked by the labeled detection oligos (DO*), preferably around 20 nucleotides. Preferably, the detection oligos satisfy two conditions:
1. The recognition sequence of the DO* is complementary to the analyte sequence and not in conflict with the capture oligo nucleotides. The capture and detection oligo nucleotides are preferably chosen such that they cover a continuous strand of the analyte mRNA without any gaps. This improves specificity and stability of the system.
2. A region different from the recognition sequence of the detection oligo includes one or more dye molecules symbolised as *.

Upon hybridization of the capture and detection oligos to the analyte mRNA and concomitant binding of the analytes to the beads the thus created system can be detected in particular by using a confocal fluorescence imaging instrument. Since the beads are chosen such that they sediment on the well bottom the 3-dimensional analyte volume was effectively reduced to a 2-dimensional detection volume.

The sequence of the quencher oligonucleotides for recognition of the detection oligos is at least partially complementary to the sequence of the detection oligos.

## Claims

1. A method for detecting in a mixture an amount of analytes with probes specifically interacting with the analytes
• the probe having a first reporter unit reporting of binding of the probe to the analyte,
• the analyte is bound to a solid support,
• the solid support is labelled with a second reporter unit,
• the method comprising the steps of
• contacting a sample containing the analyte with the solid support,
• contacting the analyte with the probe having the first reporter unit,
• measuring the binding of the probe with the analyte via the first reporter unit, and
• considering a signal due to the second reporter unit which is labelling the support.

2. The method of claim 1 wherein a quencher is added.

3. The method according to claim 1 wherein the first and/or the second reporter unit is a dye.

4. The method according to claim 3 wherein the first and/or second reporter unit is a fluorescent or chemiluminescent dye.

5. The method of claim 3 wherein the dye of first reporter unit is different from the dye of the second reporter unit.

6. The method of claim 1 wherein the analytes are proteins, nucleic acids or low molecular compounds.

7. A homogeneous assay for detecting analytes in a sample by using
• detection oligo nucleotides being labelled with a dye which reports binding of the detetion oligo nucleotide (DO) to the analytes,
• capture oligo nucleotides bound to a solid support, the capture oligo nucleotides (CO) concentrating the analyte at the surface of the solid support,
• the method comprising the steps of contacting a sample with the detection oligo nucleotides and the solid support having capture oligo nucleotides, and measuring the amount of detection oligo nucleotides bound to the analyte which is concentrated at the surface of the solid support.

8. The method according to claim 7 wherein the solid support is labelled with a dye different from the dye of the detection oligo nucleotide.

9. The method according to claims 7 and/or 8 wherein the dyes are luminescent dyes, preferably fluorescent dyes.

10. The method according to claim 9 wherein a quencher oligo nucleotide is added in order to reduce the fluorescence background.

11. The method according to at least one of claims 7 to 10 wherein the analytes are proteins or nucleic acids.

12. The method of at least one of the claims 1 to 11 wherein the solid support are beads, cells, bacteria.

13. The method of at least one of the claims 1 to 12 wherein the analyte is bound to the support by a capture unit, in particular a capture oligo nucleotide.

14. The method of claim 13 wherein the capture unit comprises a first portion bound to the support and a second portion capable of binding the analyte.

15. The method of claim 14 wherein the first portion of the capture unit is covalently bound to the support.

16. The method of claim 15 wherein the first portion of the capture unit is bound to the support by affinity interaction such as streptavidin/biotin interaction.

17. The method of at least one of the claims 13 to 16 wherein the first portion of the capture unit is biotinyl and the support is at least partially coated with streptavidin.

18. The method of at least one of the claims 13 to 17 wherein the analyte is mRNA and the second portion of the capture unit is an olig nucleotide hybridizing with the mRNA.

19. The method of claim 6 wherein the first reporter unit comprises an oligo nucleotide hybridizing with the nucleic acid analyte.

20. The method of claim 19 wherein the first reporter unit is an oligo nucleotide hybridizing with the oligo nucleotide analyte the reporter unit having covalently bound a fluorescent dye which fluorescence is quenched upon hybridizing with the nucleic acid analyte.

21. The method of claim 20 wherein the dye is Fluorescein, Rhodamine Green, Tetramethylrhodamine (TAMRA), Cy3 or rhodamine-6-G.

22. The method of claim 21 wherein the fluorescence of unbound first reporter unit is quenched with a quencher unit.

23. The method of at least one of the claims 1 to 22 wherein the second reporter unit is bound to the support covalently or by affinity interaction.

24. The method of claim 23 wherein the second reporter unit is biotinylated and is attached to the support via the streptavidin which is linked to the support.

25. The method of claim 24 wherein the second reporter unit is labelled with the dye MR 121, Cy5, Texas Red or EvoBlue30.

26. Use of the method of at least one of the claims 1 to 25 in High Throughput Screening (HTS).
